Europäisches Patentamt

(19) European Patent Office

Office européen des brevets

(11) Numéro de publication: **0 244 315**
**B1**

(12) **FASCICULE DE BREVET EUROPEEN**

(45) Date de publication du fascicule du brevet: **05.09.90**

(51) Int. Cl.⁵: **A 61 K 9/08**, A 61 K 47/00

(21) Numéro de dépôt: **87400964.0**

(22) Date de dépôt: **24.04.87**

(54) Procédé de préparation d'une solution pharmaceutique aqueuse d'un principe actif constitué par un acide organique.

(30) Priorité: **30.04.86 FR 8606346**

(43) Date de publication de la demande:
**04.11.87 Bulletin 87/45**

(45) Mention de la délivrance du brevet:
**05.09.90 Bulletin 90/36**

(84) Etats contractants désignés:
**AT BE CH DE ES FR GB GR IT LI LU NL SE**

(56) Documents cités:
**EP-A-0 076 658**
**DE-A-2 428 226**
**DE-A-3 507 024**
**GB-A-2 059 768**
**US-A-3 193 459**

**CHEMICAL ABSTRACTS, vol. 99, 1983, page 342, no. 200541h, Columbus, Ohio, US; & JP-A-58 152 811 (SUMITOMO CHEMICAL CO., LTD.) 10-09-1983**

(73) Titulaire: **LABORATOIRES CHAUVIN S.A.**
**104 Rue de la Galéra**
**F-34000 Montpellier (FR)**

(72) Inventeur: **Maurin, Florence**
**2 Lotissement La Colline Vailhauquès**
**F-34570 Pignan (FR)**
Inventeur: **Coquelet, Claude**
**113, rue des Lilas**
**F-34980 St. Gely du Fese (FR)**
Inventeur: **Tournoux, Alain**
**Coteau Fleuri 1865 rue de l'Aigue longue**
**F-34100 Montpellier (FR)**

(74) Mandataire: **Moncheny, Michel et al**
**c/o Cabinet Lavoix 2 Place d'Estienne d'Orves**
**F-75441 Paris Cedex 09 (FR)**

EP 0 244 315 B1

Courier Press, Leamington Spa, England.

## Description

La presente invention concerne un procédé de preparation d'une solution pharmaceutique aqueuse d'un principe actif constitué par un acide organique considéré normalement comme très peu soluble ou insoluble dans l'eau.

La présente invention vise plus particulièrement un procédé de preparation d'une solution pharmaceutique aqueuse qui contienne des quantités notables d'un tel principe actif (généralement de 0,05 à 5% en poids) et qui soit stable pendant au moins un mois dans les conditions normales d'utilisation. Elle concerne en particulier un procédé de préparation de solutions ophtalmiques.

Diverses tentatives ont déjà été faites pour solubiliser des principes actifs très peu solubles ou insolubles dans l'eau. On peut citer notamment les méthodes suivantes:

1) La solubilisation micellaire

Le système de micellisation permet la solubilisation dans l'eau de substances hydrophobes mais le principe actif ainsi dissous risque d'être beaucoup moins stable (altération ou hydrolyse) que dans les systèmes où il est insoluble. De plus le principe actif est susceptible de perdre une partie de son activité biologique. Ce système ne résout donc pas le problème pharmaceutique général d'administration d'un produit insoluble dans l'eau dans les conditions normales.

2) La solubilisation par co-solvant (voir par exemple la demande PCT WO 85/04106)

Les produits hydrophobes sont susceptibles d'être solubilisés par des co-solvants de type polyéthylèneglycol (PEG) de poids moléculaires divers ou autres solvants similaires, mais le principe actif est alors susceptible de former des complexes be type esters de PEG ou de s'hydrolyser par les traces d'eau présentes dans le co-solvant, ne garantissant donc pas la stabilité requise pour la formulation d'une spécialité pharmaceutique.

3) La mise en suspension du principe actif (voir par exemple le brevet US 4 087 538).

La stabilisation d'une suspension d'un principe actif sous forme micronisée est obtenue par la présence d'un agent de mise en suspension, à savoir un ou plusieurs tensio-actifs de type monooléate de sorbitan polyoxyéthyléné (Tween 80) ou autres esters de sorbitan, particulièrement d'acide laurique et stéarique, ce qui permet à la fraction solubilisée par l'eau de se micelliser. De telles suspensions, par la présence des particules micronisées provoquent une intolérance oculaire et ne conviennent pas dans le domaine ophtalmique.

Comme technique antérieure, on peut en outre citer GB—A—2 059 768 qui décrit la dissolution d'indométacine en présence d'un constituant hydrophile tel que le Tris et DE—A—2 428 226 qui décrit la lyophilisation d'une solution acide et son incorporation dans un mélange tampon.

La présente invention vise à remédier aux inconvénients exposés ci-dessus.

La présente invention est basée sur la découverte qu'il est possible de dissoudre un acide organique normalement très peu soluble dans l'eau tel que l'indométacine dans une solution aqueuse contenant une quantité suffisante d'au moins la partie basique d'un mélange tampon et que la quantité d'acide organique que l'on peut dissoudre est d'autant plus grande que la quantité de la partie basique du mélange tampon est importante.

La présente invention a ainsi pour objet un procédé de préparation d'une solution pharmaceutique aqueuse d'un principe actif constitué par un acide organique choisi parmi des composés aryliques ou hétérocycliques à groupe acide carboxylique, caractérisé en ce que

a) on dissout l'acide organique dans une première solution aqueuse contenant une quantité suffisante d'au moins une fraction de la partie basique d'un mélange tampon,

b) on lyophilise cette solution pour obtenir un produit a l'état sec et

c) au moment de l'emploi on dissout le produit a l'état sec dans une deuxième solution aqueuse contenant le reste du mélange tampon, ce reste étant tel que le pH de la solution résultant soit à une valeur physiologiquement acceptable.

Ainsi dans la première étape du procédé selon la présente invention on dissout l'acide organique dans une première solution aqueuse contenant une quantité suffisante d'au moins une fraction de la partie basique du melange tampon. Cette première solution contient généralement également une fraction de la partie acide du mélange tampon de façon à être au voisinage de la neutralité et éviter des pH trop basiques susceptibles d'affecter la stabilité du principe actif, tandis que la deuxième solution contient le reste de la partie acide du mélange tampon et peut également contenir une fraction de la partie basique du mélange tampon.

A la limite, la première solution pourrait même contenir la totalité des constituants du mélange tampon. Toutefois dans ce cas la solubilité de l'acide organique reste assez faible. En outre il est avantageux de pouvoir ajuster le pH de la solution finale à la valeur souhaitée pour l'administration grâce à la présence du reste de la partie acide du mélange tampon dans la deuxième solution. Généralement des pH voisins de la neutralité (pH de 6,5 à 7,5) sont souhaitables pour l'administration chez l'homme et les animaux.

Les mélanges tampon que l'on peut utiliser dans la présente invention sont notamment les suivants

—Tampon acide borique-borate de sodium

—Tampon phosphate monosodique-phosphate disodique

—Tampon phosphate monopotassique-phosphate dipotassique

—Tampon acide acétique-acétate de sodium

—Tampon acide citrique-citrate de sodium

et des mélanges de ces tampons. Les trois prémiers tampons sont préférés.

Pour diminuer la quantité globale du mélange tampon, on peut ajouter à la première solution des co-solvants volatils tels que l'éthanol. Une telle addition d'éthanol (ne dépassant pas généralement 10% en volume) est intéressante surtout lorsque'on veut obtenir des solutions à lyophiliser ayant des concentrations importantes en principe actif (par exemple de plus de 2% en poids).

La première solution peut en outre contenir tout agent antibactérien susceptible de garantir la qualité bactériologique des solutions, comme par exemple les esters de l'acide parahydroxybenzoïque, et/ou tout stabilisant de type antioxydant comme les sulfites de sodium et dérivés, ou l'acide édétique et ses sels, ainsi que tout autre principe actif soluble dans l'eau susceptible de completer ou d'améliorer l'activité thérapuetique du premier. En outre pour les applications ophtalmiques la solution peut contenir tout produit susceptible de prolonger le temps de contact et/ou d'améliorer la pénétration cornéene du principe actif, et notamment des composés de type dextran, dérivés cellulosiques ou d'autres viscosifiants compatibles avec la forme ophtalmique de la spécialité.

La solution ainsi préparée est suffisamment stable pour permettre sa lyophilisation, qui est effectuée selon des méthodes habituelles.

La lyophilisation présente l'avantage de fournir un produit qui peut se conserver longtemps. L'absence d'eau et de tout co-solvant tel qu'un polyéthylèneglycol évite en effet toute dégradation ou modification du principe actif. En outre les co-solvants qui peuvent être utilisés sont éliminés par sublimation au cours de la lyophilisation, de sorte que ces co-solvants ne gènent pas même pour une application ophtalmique.

Lors de l'emploi on dissout le produit lyophilisé dans la deuxième solution contenant le reste du mélange tampon, c'est-à-dire généralement le reste de la partie acide du tampon.

Cette deuxième solution comprend généralement de 75 à 98% en poids d'eau. Elle peut contenir tout antibactérien hydrosoluble, toute substance antioxydante hydrosoluble; tout autre principe actif soluble et stable dans l'eau; toute substance susceptible de stabiliser le pH à une valeur compatible avec la tolerance oculaire: tout produit ayant une influence sur la tonicité de la solution comme le chlorure de sodium, le glycocolle, le glucose; toute substance susceptible de stabiliser le principe actif en solution aqueuse. Comme stabilisants on peut utiliser notamment des polyéthylèneglycols dont la masse moleculaire peut être de 200 à 1500 et notamment de 200 à 600, à raison de 0 à 25% en poids.

Les risques de modification du principe actif par le PEG contenu dans la deuxième solution, et ce après reconstitution, sont alors bien moins importants que pour une solution dans du PEG pur du fait de la forte proportion en eau dans la deuxième solution. On peut également utiliser d'autres stabilisants tels que le propylène glycol, le tétrahydrofurfuryl polyéthylèneglycol.

La présente invention s'applique à la préparation de solutions aqueuses de principes actifs constituées par des acides organiques choisis parmi des composés aryliques ou hétérocycliques a groupe acide carboxylique tels que l'indométacine, l'aspirine, l'acide niflumique, l'ibuprofène, le pranoprofène, le kétoprofène, l'alminoprofène, le naproxène, le sulindac.

La présente invention trouve une application particulièrement intéressante dans la préparation des solutions ophtalmiques aqueuses d'indométacine.

Dans le cas de solution ophtalmiques aqueuses d'indométacine on prépare avantageusement dans un premier stade une solution contenant de 0,1 à 7,5% en poids d'indométacine et une quantité suffisante d'une fraction d'un mélange tampon contenant au moins une fraction de la partie basique du mélange tampon et généralement une fraction de la partie acide de ce mélange, cette solution ayant un pH de 6,5 à 7,5 et de préférence de 6,8 à 7,2. Un tel pH est favorable à la stabilité de l'indométacine. On peut ajouter un co-solvant tel que l'éthanol en une quantité pouvant aller jusqu'à 10% en poids lorsque la concentration en indométacine est supérieure à 2% en poids dans la solution. On lyophilise cette solution et on prépare par ailleurs une deuxième solution contenant le reste du mélange tampon. Cette deuxième solution a généralement un pH de 4,5 à 6,5 et permet d'obtenir par dissolution du produit lyophilisé dans cette deuxième solution, une solution résultante ayant un pH final compatible avec l'oeil, généralement de 6,5 à 7,5 et avantageusement de 6,8 à 7,3. Cette solution ophtalmique peut généralement avoir une concentration en indométacine de 0,05 à 1% en poids.

Il est à noter qui si on utilise comme tampon le tampon acide borique/borate de sodium la quantité en poids d'indométacine que l'on peut dissoudre dans la première solution est sensiblement égale à la quantité (en poids) de borate de sodium.

La présente invention s'applique non seulement à la préparation de solutions ophtalmiques mais également à la préparation de solutions nasales, de solutions injectables et de solutions buvables.

Les exemples suivants illustrent la présente invention.

Exemple 1

Préparation d'un collyre contenant 0,1% en poids d'indométacine et un tampon borate.

On prépare une première solution d'indométacine par agitation à la température ambiante des constituants suivants:

| | |
|---|---|
| indométacine | 0,250 g |
| borate de sodium | 0,255 g |

| acide borique | 1,405 g |
|---|---|
| dextran | 1,000 g |
| eau qsp | 100,000 ml |

Cette solution à un pH de 7,0.

On lyophilisé immédiatement cette solution après l'avoir répartie dans des flacons à raison de 2 ml de solution par flacon.

On prépare par ailleurs une deuxième solution par mélange à la température ambiante les constituants suivants:

| acide borique | 1,149 g |
|---|---|
| nipagine (conservateur) | 0,050 g |
| PEG 400 (stabilisant) | 24,850 g |
| eau qsp | 100,000 ml |

Le pH de cette solution est de 5,65.

Cette solution est répartie dans des flacons à raison de 5 ml per flacon.

Lors de l'utilisation on ajoute au produit lyophilisé contenu dans un flacon les 5 ml de la deuxième solution contenus dans un flacon de solution. On obtient un collyre contenant 0,1% en poids d'indométacine et ayant un pH de 7,1.

Cette solution à de façon remarquable une durée de conservation d'au moins un mois à température ambiante.

Exemple 2

Préparation d'un collyre contenant 0,1% en poids d'indométacine

On opère comme à l'exemple 1 en utilisant les solutions suivantes:

—Première solution (à lyophiliser) répartie à raison de 2 ml par flacon

| indométacine | 0,250 g |
|---|---|
| dextran | 1,000 g |
| phosphate monosodique | 0,612 g |
| phosphate disodique | 6,400 g |
| nipagine | 0,125 g |
| eau qsp | 100,000 ml |
| pH | 7,25 |

—Deuxième solution, répartie à raison de 5 ml par flacon

| phosphate monosodique | 0,874 g |
|---|---|
| PEG 400 | 10,000 g |
| eau qsp | 100,000 ml |
| pH | 5,1 |

Le collyre final à un pH de 7,1 et est stable pendant au moins un mois.

Exemple 3

Préparation d'un collyre contenant 0,4% en poids d'indométacine

On opère comme à l'exemple 1 en utilisant les solutions suivantes:

—Première solution (à lyophiliser) répartie à raison de 2 ml par flacon

| indométacine | 1,000 g |
|---|---|

| dextran | 1,000 g |
|---|---|
| acide borique | 2,180 g |
| borate de sodium | 1,000 g |
| eau qsp | 100,000 ml |
| pH | 7,0 |

—Deuxième solution (répartie à raison de 5 ml par flacon)

| acide borique | 1,500 g |
|---|---|
| PEG 400 | 25,000 g |
| eau qsp | 100,000 ml |
| pH | 5,55 |

Le collyre final à un pH de 7,1 et est stable pendant au moins deux mois.

Exemple 4

Préparation d'un collyre contenant 0,1% en poids d'ibuprofène

On opère comme à l'exemple 1 en utilisant les solutions suivantes:

—Première solution (à lyophiliser) répartie à raison de 2 ml par flacon

| ibuprofène (acide) | 0,250 g |
|---|---|
| nipagine | 0,125 g |
| acide borique | 2,190 g |
| borate de sodium | 0,500 g |
| dextran | 1,000 g |
| eau qsp | 100,000 ml |
| pH | 7,0 |

—Deuxième solution (répartie à raison de 5 ml de flacon)

| acide borique | 1,224 g |
|---|---|
| PEG 400 | 25,000 g |
| eau qsp | 100,000 ml |
| pH | 5,6 |

Le collyre final à un pH de 7,0 et est stable pendant au moins un mois.

Exemple 5

Collyre contenant 0,1% en poids d'indométacine

On opère comme à l'exemple 1.

—Première solution:

| indométacine | 0,250 g |
|---|---|
| éthanol | 5,000 ml |
| borate de sodium | 0,1315 g |
| acide borique | 0,450 g |
| nipagine | 0,125 g |
| dextran | 5,000 g |
| eau qsp | 100,000 ml |
| pH | 7,0 |

—Deuxième solution:

| borate de sodium | 0,018 g |
|---|---|
| acide borique | 0,727 g |
| PEG 400 | 24,85 g |
| eau qsp | 100,000 ml |
| pH | 7,20 |
| pH du collyre final | 7,2 |

Exemple 6
Collyre contenant 0,4% en poids d'indométacine
On opère comme à l'exemple 1.

—Première solution:

| | |
|---|---|
| indométacine | 1,000 g |
| borate de sodium | 1,000 g |
| acide borique | 2,180 g |
| dextran | 1,000 g |
| eau qsp | 100,000 ml |
| pH | 7 |

—Première solution:

| | |
|---|---|
| acide borique | 1,500 g |
| nipagine | 0,050 g |
| PEG 600 | 25,000 g |
| eau qsp | 100,000 ml |
| pH | 5,5 |
| pH du collyre final | 7,0 |

Exemple 7
Collyre contenant 0,1% en poids d'indométacine
On opère comme à l'exemple 1.

—Première solution:

| | |
|---|---|
| indométacine | 0,250 g |
| borate de sodium | 0,255 g |
| acide borique | 1,405 g |
| dextran | 1,000 g |
| eau qsp | 100,000 ml |
| pH | 7,0 |

—Deuxième solution:

| | |
|---|---|
| acide borique | 1,149 g |
| nipagine | 0,050 g |
| PEG 400 | 5,000 g |
| eau qsp | 100,000 ml |
| pH | 5,7 |
| pH du collyre final | 7,0 |

Exemple 8
Collyre contenant 2% en poids d'indométacine
On opère comme à l'exemple 1.

—Première solution:

| | |
|---|---|
| indométacine | 5,000 g |
| éthanol | 5,000 ml |
| borate de sodium | 5,000 g |
| acide borique | 2,140 g |
| dextran | 1,000 g |
| eau qsp | 100,000 ml |
| pH | 7,2 |

—Deuxième solution:

| | |
|---|---|
| acide borique | 2,452 g |
| PEG 400 | 25,000 g |
| nipagine | 0,050 g |
| eau qsp | 100,000 ml |
| pH | 5,2 |
| pH du collyre final | 7,00 |

Exemple 9
Préparation d'un collyre contenant 0,1% d'indométacine

On opère comme à l'exemple 1 de façon à obtenir par flacon la formule suivante du lyophili-sat.

| | |
|---|---|
| indométacine | 5 mg |
| borax | 5,1 mg |
| acide borique | 28,096 mg |
| dextran | 20 mg |

On prépare par ailleurs a solution suivante:

| | |
|---|---|
| PEG 400 | 1242,5 mg |
| nipagine | 2,5 mg |
| EDTA | 2,5 mg |
| acide borique | 99,75 mg |
| borax | 7,45 mg |
| eau purifiée qsp | 5 ml |

Lors de l'utilisation on ajoute au lyophilisat la solution constituant le solvant et l'on obtient un collyre reconstitué ayant la formule centésimale suivante:

| | |
|---|---|
| indométacine | 0,1 g |
| borax | 0,251 g |
| acide borique | 2,5569 g |
| nipagine | 0,05 g |
| EDTA | 0,05 g |
| PEG 400 | 24,85 g |
| dextran | 0,4 g |
| eau purifiée qsp | 100 ml |

**Revendications**

1. Procédé de préparation d'une solution pharmaceutique aqueuse d'un principe actif constitué par un acide organique choisi parmi des composés aryliques ou hétérocycliques à groupe acide carboxylique, caractérisé en ce que
a) on dissout l'acide organique dans une première solution aqueuse contenant une quantité suffisante d'au moins une fraction de la partie basique d'un mélange tampon,
b) on lyophilise cette solution pour obtenir un produit à l'état sec et
c) au moment de l'emploi on dissout le produit à l'état sec dans une deuxième solution aqueuse contenant le reste du mélange tampon, ce reste étant tel que le pH de la solution résultante soit à une valeur physiologiquement acceptable.

2. Procédé selon la revendication 1, caractérisé en ce que la première solution contient également une fraction de la partie acide du mélange tampon et la deuxième solution contient le reste du mélange tampon.

3. Procédé selon la revendication 1 ou la revendication 2, caractérisé en ce que les mélanges tampons sont choisis parmi les tampons acide borique-borate de sodium, phosphate monosodique-phosphate disodique, phosphate monopotassique-phosphate dipotassique, acide acétique-acétate de sodium et acide citrique-citrate de sodium.

4. Procédé selon l'une quelconque des revendications 1 à 3, caractérisé en ce que la première solution contient un co-solvant volatil.

5. Procédé selon la revendication 4, caractérisé en ce que le co-solvant est l'éthanol.

6. Procédé selon l'une quelconque des revendications 1 à 5, caractérisé en ce que la deuxième solution contient un stabilisant.

7. Procédé selon la revendication 6, caractérisé en ce que le stabilisant est un polyéthylèneglycol dont la masse moléculaire est de 200 à 1500.

8. Procédé selon l'une quelconque des revendications 1 à 7, pour la préparation d'une solution ophtalmique d'indométacine, caractérisé en ce que

a) on prépare une première solution contenant 0,1 à 7,5% en poids d'indométacine et une quantité suffisante d'au moins une fraction de la partie basique d'un mélange tampon, cette solution ayant un pH de 6,5 à 7,5,

b) on lyophilise cette solution pour obtenir un produit à l'état sec,

c) au moment de l'emploi on dissout le produit à l'état sec dans une deuxième solution contenant le reste du mélange tampon, ce reste étant tel que le pH de la solution résultante soit de 6,5 à 7,5.

## Patentansprüche

1. Verfahren zur Herstellung einer wäßrigen pharmazeutischen Lösung eines Wirkstoffs, bestehend aus einer organischen Säure, die ausgewählt ist aus Aryl- oder heterocyclischen Verbindungen mit Carbonsäuregruppe, dadurch gekennzeichnet, daß man

a) die organische Säure in einer ersten wäßrigen Lösung, die eine ausreichende Menge von wenigstens einem Teil des basischen Anteils einer Puffermischung enthält, löst,

b) diese Lösung lyophilisiert, um ein Produkt in trockenem Zustand zu erhalten und

c) im Augenblick der Verwendung das Produkt in trockenem Zustand in einer zweiten wäßrigen Lösung, die den Rest der Puffermischung enthält, löst, wobei der Rest so beschaffen ist, daß der pH der resultierenden Lösung bei einem physiologisch annehmbaren Wert liegt.

2. Verfahren nach Anspruch 1, dadurch gekennzeichnet, daß die erste Lösung auch einen Teil des sauren Anteils der Puffermischung enthält und die zweite Lösung den Rest der Puffermischung enthält.

3. Verfahren nach Anspruch 1 oder Anspruch 2, dadurch gekennzeichnet, daß die Puffermischungen ausgewählt sind aus Borsäure-Natriumborat, Natriumdihydrogenphosphat-Dinatriumhydrogenphosphat, Kaliumdihydrogenphosphat-Dikaliumhydrogenphosphat, Essigsäure-Natriumacetat und Citronensäure-Natriumcitrat.

4. Verfahren nach irgendeinem der Ansprüche 1 bis 3, dadurch gekennzeichnet, daß die erste Lösung ein flüchtiges Co-Lösungsmittel enthält.

5. Verfahren nach Anspruch 4, dadurch gekennzeichnet, daß das Co-Lösungsmittel Ethanol ist.

6. Verfahren nach irgendeinem der Ansprüche 1 bis 5, dadurch gekennzeichnet, daß die zweite Lösung einen Stabilisator enthält.

7. Verfahren nach Anspruch 6, dadurch gekennzeichnet, daß der Stabilisator ein Polyethylenglykol ist, dessen Molekulargewicht 200 bis 1500 beträgt.

8. Verfahren nach irgendeinem der Ansprüche 1 bis 7 zur Herstellung einer ophtalmischen Lösung von Indometacin, dadurch gekennzeichnet, daß man

a) eine erste Lösung, die 0,1 bis 7,5 Gewichtsprozent Indometacin und eine ausreichende Menge von wenigstens einem Teil des basischen Anteils einer Puffermischung enthält, herstellt, wobei die Lösung einen pH von 6,5 bis 7,5 aufweist,

b) diese Lösung lyophilisiert, um ein Produkt in trockenem Zustand zu erhalten,

c) im Augenblick der Verwendung das Produkt in trockenem Zustand in einer zweiten Lösung, die den Rest der Puffermischung enthält, löst, wobei der Rest so beschaffen ist, daß der pH der resultierenden Lösung 6,5 bis 7,5 beträgt.

## Claims

1. Process for the preparation of an aqueous pharmaceutical solution containing an active ingredient constituted by an organic acid, in particular aryl or heterocyclic compounds with a carboxyl group, characterised in that,

a) the organic acid is dissolved in a first aqueous solution containing a sufficient quantity of at least one part of the basic portion of a buffer mixture,

b) the resultant solution is lyophilized in order to obtain a product in the dry state, and

c) at the time of use the dry state product is dissolved in a second aqueous solution containing the remainder of the buffer mixture, this remainder being such that the pH of the resultant solution must have a physiologically-acceptable value.

2. Process according to claim 1, characterised in that the first solution also contains a part of the acid portion of the buffer mixture, and the second solution contains the remainder of the buffer mixture.

3. Process according to claim 1 or claim 2, characterised in that the buffer mixtures are selected from the boric acid-sodium borate, monosidum phosphate-disodium phosphate, monopotassium phosphate-dipotassium phosphate, acetic acid-sodium acetate and citric acid-sodium citrate buffer mixtures.

4. Process according to any of claims 1 to 3, characterised in that the first solution contains a volatile co-solvent.

5. Process according to claim 4, characterised in that the co-solvent is ethanol.

6. Process according to any of claims 1 to 5, characterised in that the second solution contains a stabilizer.

7. Process according to claim 6, characterised in that the stabilizer is a polyethyleneglycol having a molecular weight of 200 to 1500.

8. Process according to any of claims 1 to 7, for the preparation of an ophthalmic solution of indomethacin, characterised in that:

a) a first solution is prepared containing 0.1 to 7.5% by weight of indomethacin and a sufficient quantity of at least one part of the basic portion of the buffer mixture, this solution having a pH value of 6.5 to 7.5,

b) the resultant solution is lyophilized in order to obtain a dry state product, and

c) at the time of use, the dry state product is redissolved in the second solution which contains the remainder of the buffer mixture, this remainder being such that the pH of the resultant solution is of 6.5 to 7.5.